# FASCICULE DE BREVET EUROPEEN

(11) **EP 0 706 348 B1**
(45) Date de publication et mention de la délivrance du brevet: **05.03.1997**
(21) Numéro de dépôt: 94920993.6
(22) Date de dépôt: 29.06.1994
(51) Int. Cl.: A61C 1/18, A61C 1/05, A61M 3/02

(54) **INSTRUMENT CHIRURGICAL, DESTINE NOTAMMENT A LA CHIRURGIE DENTAIRE**
CHIRURGISCHES GERÄT, INSBESONDERE FÜR DIE ZAHNCHIRURGIE
SURGICAL INSTRUMENT, IN PARTICULAR FOR DENTAL SURGERY

(30) Priorité: 29.06.1993 FR 9307921; 14.01.1994 FR 9400385
(43) Date de publication de la demande: 17.04.1996
(73) Titulaire: SATELEC S.A., F-33700 Merignac (FR)
(72) Inventeur: PINEL, Alain, F-33127 Martignas-sur-Jalles (FR); DUPEYRON, Pascal, F-33000 Bordeaux (FR); DIERAS, Francis, F-33000 Bordeaux (FR)
(74) Mandataire: Bruder, Michel
(86) Numéro de dépôt international: FR9400791
(87) Numéro de publication internationale: WO9501136

(56) Documents cités:
- EP-A- 0 125 784
- DE-A- 3 407 199
- US-A- 2 543 075
- US-A- 5 054 584

## Description

La présente invention concerne un instrument chirurgical, destiné notamment à la chirurgie dentaire, et plus particulièrement un instrument chirurgical alimenté en au moins un fluide stérile.

Les instruments utilisés en chirurgie dentaire, tels que ceux faisant par exemple appel aux ultrasons, nécessitent la plupart du temps, d'une part, une alimentation en énergie, à savoir soit une énergie électrique soit une énergie fournie par un gaz sous pression et, d'autre part, une alimentation en liquide tel qu'un liquide d'irrigation.

On sait que certaines interventions parodontiques utilisant ce type d'instruments, et notamment celles se déroulant entre la gencive et la dent d'un patient, telles que les interventions de surfaçage radiculaire, doivent être effectuées avec des instruments et des fluides d'irrigation stériles. Si les instruments odontologiques actuels permettent de remplacer facilement et rapidement un élément porte-outil, ou pièce à main, non stérile, par un élément porte-outil stérile, ils ne permettent pas, par contre, d'échanger avec la même facilité le fluide d'irrigation qui alimente cet instrument pour le remplacer par un fluide stérile. Dans la pratique courante, le chirurgien ayant débute une intervention dans une configuration non stérile et souhaitant poursuivre celle-ci en configuration stérile est ainsi contraint de changer la totalité de son instrument, ce qui représente pour lui une contrainte supplémentaire.

Un instrument chirurgical comme décrit dans le préambule de la revendication 1 est connu du document DE-A-3 407 199

La présente invention a pour but de proposer un moyen permettant au chirurgien de passer, de façon facile et quasi instantanée, d'une configuration non stérile à une configuration stérile, ou d'un liquide d'irrigation donné à un autre liquide d'irrigation.

La présente invention a ainsi pour objet un instrument chirurgical et notamment un instrument chirurgical alimenté en au moins un fluide stérile, comprenant un élément actif et un élément d'alimentation, la partie postérieure de l'élément actif et la partie antérieure de l'élément d'alimentation comportant des moyens de connexion complémentaires permettant d'assurer leur solidarisation de façon étanche, l'élément d'alimentation comportant au moins une canalisation d'alimentation en un premier fluide, débouchant à l'endroit des moyens de connexion, et l'élément actif comprend des moyens d'alimentation en au moins un second fluide, caractérisé en ce qu'il comporte des moyens assurant l'obturation de la susdite canalisation d'alimentation en un premier fluide, lorsque l'élément actif est connecté à l'élément d'alimentation.

Dans un mode de mise en oeuvre de l'invention, les moyens assurant l'obturation de la susdite canalisation d'alimentation en un premier fluide, lorsque l'élément actif est connecté à l'élément d'alimentation, font partie intégrante de la partie postérieure de l'élément actif.

Dans un autre mode de mise en oeuvre particulièrement intéressant de l'invention, l'élément actif est constitué d'au moins deux éléments connectables entre eux de façon étanche, à savoir un élément antérieur, ou tête, et un élément postérieur, ou connecteur d'irrigation, les moyens d'alimentation en au moins un second fluide sont en communication avec le connecteur d'irrigation, et la partie postérieure de la tête comporte des moyens de connexion complémentaires de ceux de l'élément d'alimentation permettant d'assurer une solidarisation étanche avec celui-ci, le canal d'alimentation en un premier fluide étant en communication avec la tête lorsque cette dernière est connectée à l'élément d'alimentation.

La présente invention peut être mise en oeuvre sur de nombreux appareils existants, et permet ainsi à un praticien de passer de façon simple et quasi instantanée d'une configuration non stérile à une configuration stérile et, de façon plus générale, de disposer au cours d'une même intervention de plusieurs liquides d'irrigation qui peuvent ou non être mélangés et qui, de plus, sont accessibles de façon quasi immédiate.

On décrira ci-après, à titre d'exemple non limitatif, plusieurs formes d'exécution de la présente invention, en référence au dessin annexé sur lequel :
La figure 1 est une vue en coupe axiale d'un détartreur suivant l'invention.
La figure 1a est une vue en coupe axiale partielle de la pièce à main d'un détartreur suivant l'état antérieur de la technique.
La figure 2 est une vue en coupe axiale d'une seringue dentaire suivant l'invention.
La figure 2a est une vue en coupe axiale partielle d'une seringue dentaire suivant l'état antérieur de la technique.
La figure 3 est une vue en coupe axiale d'une turbine dentaire suivant l'invention.
La figure 4a est une vue en coupe axiale partielle d'un élément porte-outil, ou pièce à main, d'un instrument chirurgical destiné à des interventions odontologiques.
La figure 4b est une vue en coupe axiale suivant la ligne IVb-IVb de la figure 5, d'un connecteur d'irrigation, destiné à se connecter sur la partie postérieure de la pièce à main représentée sur la figure 4a, de façon à constituer un élément actif.
La figure 4c est une vue en coupe axiale partielle d'un élément d'alimentation destiné à se connecter sur la partie postérieure, soit de la pièce à main représentée sur la figure 4a, soit du connecteur d'irrigation représenté sur la figure 4b.
La figure 5 est une vue en coupe transversale de la pièce à main représentée sur la figure 4a suivant la ligne V-V de celle-ci.
Les figures 6, 7 et 8 sont des vues partielles, en coupe axiale, de trois variantes de mise en oeuvre de l'invention.

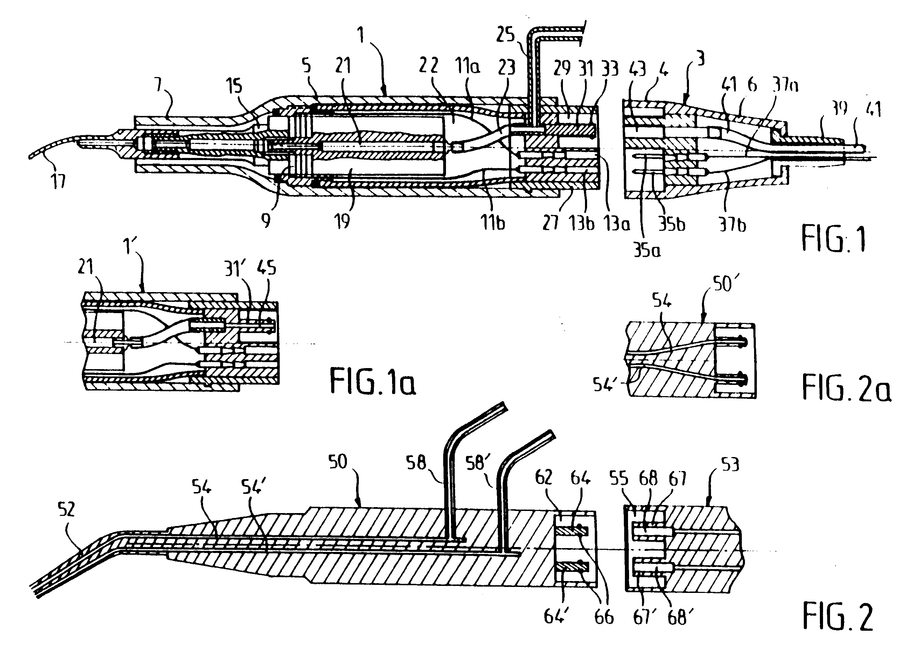

Dans un premier mode de mise en oeuvre de l'invention, illustré sur la figure 1, l'instrument chirurgical est constitué d'un appareil détartreur. Cet appareil détartreur se compose d'un élément actif formé d'une pièce à main 1 destinée à se connecter à un élément d'alimentation 3. La pièce à main 1 est constituée d'un corps creux cylindrique 5, fait d'un matériau isolant, qui se termine, sur sa partie antérieure, par une partie cylindrique creuse 7 de plus faible diamètre. Le corps 5 renferme, de façon connue, des pastilles piézo-électriques 9 dont les parties extrêmes sont respectivement reliées, par des conducteurs d'alimentation électrique 11a,11b, à des fiches femelles 13a, 13b ouvertes sur la partie postérieure de la pièce à main 1.

Un amplificateur de vibration 15 est en contact avec la face antérieure des pastilles piézo-électriques 9, cet amplificateur 15 se terminant par un insert 17 soumis à un mouvement vibratoire longitudinal lorsque les pastilles piézo-électriques 9 sont alimentées en courant alternatif de fréquence élevée.

La partie postérieure des pastilles piézo-électriques 9 est en contact avec une contremasse 19. Cette dernière est percée d'un canal axial 21 qui débouche, sur sa face postérieure, dans une cavité 22 du corps 5 et qui se prolonge vers l'avant, au travers de l'amplificateur 15 jusqu'à l'endroit de l'insert 17. L'entrée du canal 21 est en communication, par un tuyau souple 23, avec un tuyau radial rigide coudé 25, qui est solidaire du corps 5 et qui est susceptible d'être relié, notamment, à une source de liquide stérile sous pression (non représentée sur le dessin).

La partie postérieure de la pièce à main 1 se termine par un fond cylindrique 27, constitué d'un matériau isolant, qui est fixé et engagé dans la partie extrême arrière du corps 5. Le fond 27 reçoit les deux fiches femelles 13a et 13b, et est creusé d'une cavité cylindrique 29 de section transversale semi-circulaire, traversée par un bouchon obturateur cylindrique longitudinal 31 qui est terminé à son extrémité par un joint d'étanchéité torique 33.

L'élément d'alimentation 3 est formé essentiellement d'une partie antérieure cylindrique 4, de diamètre interne sensiblement égal au diamètre externe du fond 27, et d'une partie postérieure tronconique 6. La face antérieure de l'élément d'alimentation 3 comporte différentes cavités et bossages complémentaires de la partie postérieure du fond 27, de façon que l'élément d'alimentation 3 puisse venir se connecter et s'emboîter sur celui-ci. La partie antérieure de l'élément d'alimentation 3 comprend ainsi essentiellement deux broches mâles 35a, 35b, destinées à prendre respectivement place dans les fiches femelles 13a et 13b du fond 27. Ces broches mâles 35a,35b sont reliées à des fils 37a,37b d'alimentation en courant alternatif de fréquence élevée, qui sont logés dans une gaine 39 qui relie la partie postérieure de l'élément d'alimentation 3 à un générateur de courant, non représenté sur le dessin. A l'intérieur de la gaine 39 prend place également une canalisation 41, reliée à une arrivée d'eau sous pression (non représentée sur le dessin), et qui débouche dans une cavité cylindrique 43 ouverte sur la partie antérieure de l'élément d'alimentation 3, face au bouchon obturateur 31, lorsque la pièce à main 1 est prête à être connectée à l'élément d'alimentation 3. Dans ces conditions, lorsque l'élément d'alimentation 3 est connecté à la pièce à main 1, le bouchon obturateur 31 se trouve engagé dans la cavité 43 dont il assure l'obturation, et les broches mâles 35a,35b se trouvent dans les fiches femelles 13a,13b, assurant ainsi la liaison électrique des pastilles piézo-électriques 9 avec le générateur de courant. De plus, dans cette position de connexion, le bouchon obturateur 31 fermant l'arrivée d'eau sous pression provenant de la canalisation 41, l'alimentation en liquide de la pièce à main 1 se fait alors par l'intermédiaire du tuyau coudé 25, relié à cet effet à une alimentation externe notamment en liquide stérile.

L'élément d'alimentation 3 peut ainsi être connecté à plusieurs types de pièces à main, à savoir des pièces à main de type classique, c'est-à-dire suivant l'état antérieur de la technique, et à des pièces à main suivant l'invention.

A titre de comparaison, on a représenté sur la figure 1a la partie postérieure d'une pièce à main 1' de type usuel suivant l'état antérieur de la technique. Cette pièce à main 1' ne comporte pas de tube coudé d'alimentation en un second liquide d'irrigation et le bouchon obturateur est remplacé par un élément 31' creusé d'un canal d'alimentation longitudinal 45 qui met en communication, par la canalisation 41, le canal axial 21, débouchant à la partie avant du détartreur, avec la canalisation d'alimentation en premier liquide d'irrigation.

Ainsi, lorsqu'une pièce à main 1', de type usuel, est connectée à l'élément d'alimentation 3, les alimentations en liquide d'irrigation et en énergie électrique de celle-ci se font par l'intermédiaire de l'élément d'alimentation 3 alors que, lorsqu'une pièce à main 1 suivant l'invention est connectée à ce même élément d'alimentation 3, l'alimentation en liquide d'irrigation se fait par l'intermédiaire du tuyau coudé 25 relié, par exemple, à des moyens d'alimentation en liquide stérile, alors que l'alimentation électrique se fait toujours par l'intermédiaire de l'élément d'alimentation 3.

Dans ces conditions, lorsque le praticien souhaite passer d'une configuration non stérile à une configuration stérile, il lui suffit d'échanger une pièce à main 1' de type usuel, contre une pièce à main 1 suivant l'invention, et de relier cette dernière à des moyens d'alimentation en liquide stérile.

La présente invention peut également être mise en oeuvre sur d'autres types d'appareils chirurgicaux et notamment, comme représenté sur la figure 2, sur une seringue dentaire.

Sur cette figure 2, la seringue dentaire est constituée d'un élément actif, ou corps 50, et d'un élément d'alimentation 53. Le corps cylindrique 50 se termine, de façon connue, par un embout coudé 52. Cet embout 52, ainsi que le corps 50, sont traversés de deux canaux longitudinaux respectifs 54, 54' débouchant à l'extrémité de l'embout 52, et chacun de ces canaux est relié, à son extrémité interne, à un tuyau radial coudé 58, 58' solidaire du corps 50 de la seringue et faisant saillie hors de ce corps. La partie postérieure du corps 50 est creusée d'une cavité cylindrique 62 ouverte sur l'extérieur, qui renferme deux bouchons obturateurs longitudinaux 64, 64', constitués de deux broches cylindriques pourvues chacune d'un joint torique d'étanchéité 66.

Le corps 50 de la seringue dentaire est destiné à se connecter à l' élément d'alimentation 53, dont la partie antérieure est creusée, à cet effet, d'une cavité cylindrique 55, de diamètre interne sensiblement égal au diamètre externe du corps 50, et dont le fond comporte deux bossages longitudinaux en saillie 67, 67'creusés chacun d'un canal longitudinal 68, 68' d'un diamètre interne sensiblement égal à celui des bouchons obturateurs 64,64' de façon à pouvoir accueillir ceux-ci lorsque le corps 50 est connecté à l'élément d'alimentation 53. Les canaux 68,68' sont respectivement reliés à des moyens d'alimentation en liquide et en air sous pression, non représentés sur le dessin. Lorsque le corps 50 de la seringue dentaire est connecté à l'élément d'alimentation 53, les bouchons obturateurs 64,64' assurent l'obturation des canaux d'alimentation en liquide et en air sous pression 68,68'. L'alimentation en fluide de la seringue se fait alors par les tuyaux coudés 58,58', qui sont à cet effet reliés à des sources externes respectives d'alimentation en liquide et en air stériles sous pression, non représentées sur le dessin.

L'élément d'alimentation 53 peut également recevoir des éléments actifs de seringue 50' de type usuel suivant l'état antérieur de la technique, ainsi que représenté sur la figure 2a. Lorsqu'un élément actif de type usuel 50' est connecté à l'élément d'alimentation 53, les canaux d'alimentation en liquide et en air sous pression 68,68' se trouvent respectivement reliés aux canaux 54,54' de l'élément actif 50' de la seringue, dont l'embout est ainsi en mesure de fournir en sortie de l'air et/ou un liquide provenant de l'élément d'alimentation 53.

Lorsque le praticien veut passer d'une configuration de seringue non stérile à une configuration de seringue stérile, il remplace l'élément actif non stérile 50' de celle-ci par un élément actif de seringue stérile 50 suivant l'invention dont les tubes d'alimentation 58,58' sont respectivement reliés à des sources de liquide et d'air stériles.

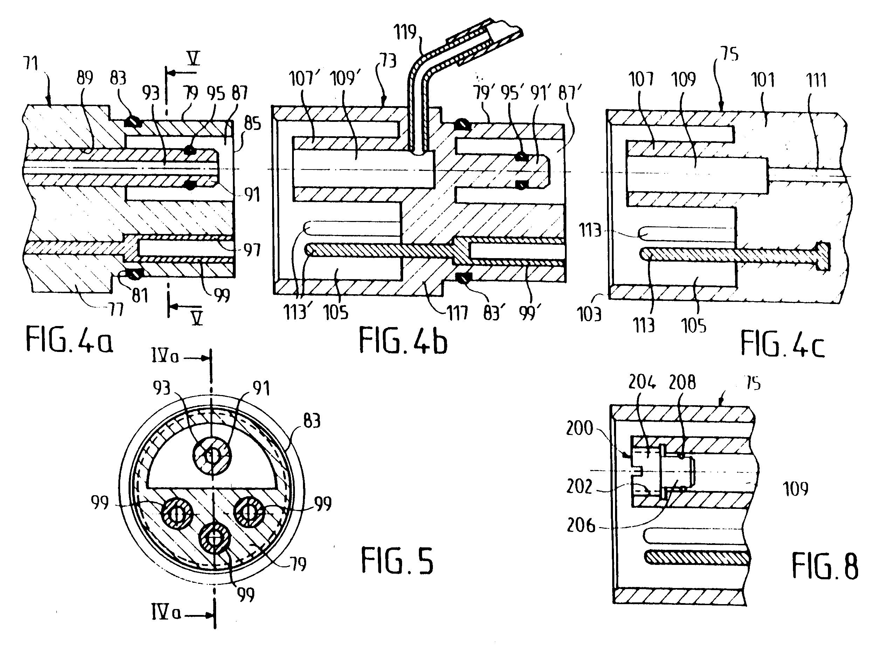

Dans une variante représentée sur la figure 3, la présente invention est mise en oeuvre sur une turbine dentaire.

Cette dernière se compose d'un élément actif ou élément porte-outil 70, qui est en mesure de se connecter à un élément d'alimentation 72. La forme globale externe de l'élément porte-outil 70 est du type de ceux de l'état antérieur de la technique. De façon schématique la turbine dentaire comprend un corps 74, de forme cylindrique, prolongé, sur sa partie antérieure, par une partie tronconique et par un élément tubulaire coudé par rapport à l'axe du corps 74 et qui supporte une tête porte-fraise 76. Le corps 74 de l'élément porte-outil 70 est creusé, dans le sens longitudinal, de deux canaux parallèles 78 et 78' qui débouchent, à l'une de leurs extrémités, dans la tête porte-fraise 76, de part et d'autre de l'arbre portant la fraise et, à l'autre extrémité, dans des tubes radiaux coudés 80, 80', destinés à être connectés à des sources respectives de liquide et de gaz stériles, non représentées sur le dessin.

Le corps 74 comporte, sensiblement au niveau de sa partie tronconique, une turbine proprement dite 84 (représentée de façon schématique sur le dessin) qui est reliée à une canalisation d'alimentation en air sous pression 86 et à une canalisation d'évacuation d'air 86', ces canalisations débouchant à l'extrémité postérieure de l'élément porte-outil 70, dans des broches creuses respectives 88, 88' qui font saillie longitudinalement sur le fond de la partie postérieure du corps 74. La partie postérieure du corps 74 est également pourvue de deux bouchons obturateurs axiaux 90, 90'.

L'élément d'alimentation 72 est de forme cylindrique et est creusé, sur sa face antérieure, de deux cavités cylindriques 92, 92', respectivement reliées, par des conduits 94, 94', à une source d'air sous pression et à une sortie d'air, et qui sont destinées à recevoir respectivement les broches creuses 88, 88'. La face antérieure de l'élément d'alimentation est également creusée de deux cavités cylindriques 96, 96', de plus petit diamètre, respectivement reliées, par des conduits 98, 98' à des moyens d'alimentation en liquide d'irrigation et en air, et qui sont prévues dans des positions leur permettant de recevoir respectivement les bouchons obturateurs 90, 90'.

Le principe de mise en oeuvre est le même que celui précédemment décrit, à savoir que la turbine dentaire décrite sur la figure 3 est utilisable en mode stérile et que, dans cette configuration, lorsque l'élément porte-outil 70 est connecté à l'élément d'alimentation 72, la turbine 84 se trouve alimentée en air sous pression par l'intermédiaire de l'élément d'alimentation 72, alors que l'alimentation en liquide et en air stérile se fait par les tubes coudés 80, 80', les cavités correspondantes 96, 96' de l'élément d'alimentation 72 étant alors obturées par les bouchons obturateurs 90, 90'. Comme dans le cas des instruments précédents, et dans une configuration non stérile, l'élément d'alimentation peut être connecté à un élément porte-outil de type usuel, toutes les alimentations en fluide provenant alors de l'élément d'alimentation 72.

Dans un mode de mise en oeuvre de l'invention, l'élément actif peut être constitué de deux éléments, à savoir une tête et un connecteur d'irrigation.

On a ainsi respectivement représenté sur les figures 4a, 4b et 4c la partie postérieure d'un élément porte-outil de type usuel, ou tête 71, un connecteur d'irrigation 73 et la partie antérieure d'un élément d'alimentation 75.

Le connecteur d'irrigation 73 est destiné à se connecter, par sa partie antérieure, sur la partie postérieure de la tête 71, si bien que ces deux éléments, une fois connectés, constituent l'élément actif de l'instrument. La partie postérieure du connecteur d'irrigation 73 est destinée à se connecter sur la partie antérieure de l'élément d'alimentation 75, et l'ensemble des trois susdits éléments constitue alors un instrument chirurgical utilisable en technique de traitement dentaire, notamment pour décoller les plaques de tartre qui se déposent sur les dents.

La tête 71 est constituée d'un corps cylindrique 77, en une matière plastique isolante, qui renferme des pastilles piézo-électriques (non représentées sur le dessin) qui, sous l'action d'un courant de fréquence élevée, soumettent un outil, ou insert, disposé à la partie antérieure de la tête, à un mouvement vibratoire longitudinal. Ces derniers éléments, de type connu, ne sont pas représentés sur le dessin.

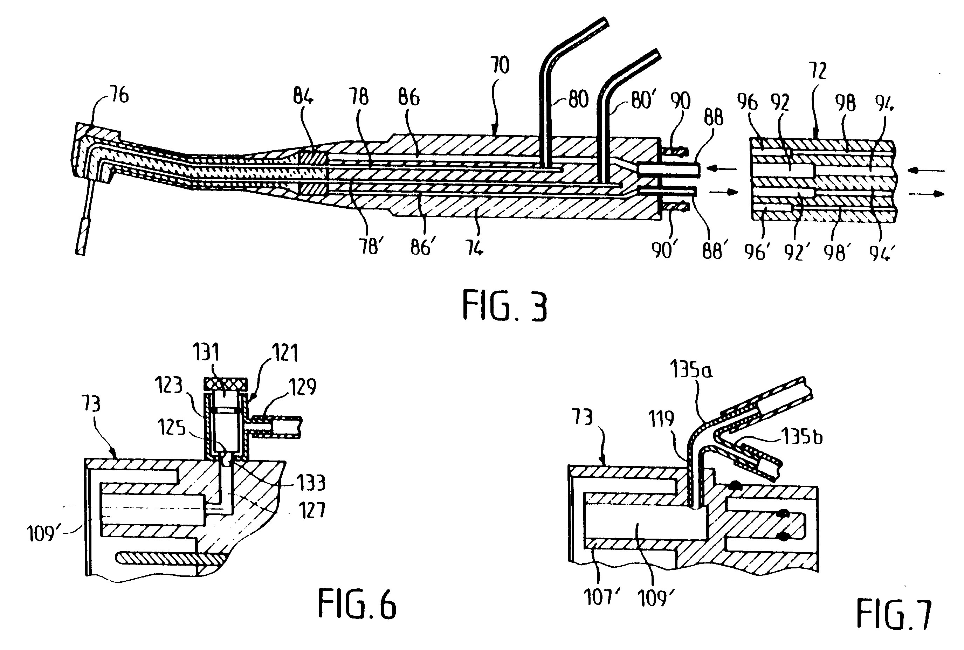

La tête 71 se termine, à sa partie postérieure, par une portion cylindrique 79, de plus petit diamètre, qui est creusée d'une rainure circulaire périphérique 81 dans laquelle est encastré un joint d'étanchéité torique 83. La face arrière 85 de la portion cylindrique 79 de la tête 71 est creusée d'un chambrage cylindrique 87, de section transversale semi-circulaire. Le fond du chambrage 87 est lui-même creusé d'un trou 89 d'axe longitudinal, dans lequel est fixé un tube métallique 91, pourvu d'un canal central 93 relié à l'extrémité antérieure de la tête 71. La périphérie postérieure du tube 91 est creusée d'une rainure circulaire dans laquelle est encastré un joint d'étanchéité torique 95. La face arrière 85 de la tête 71 est également creusée de trois logements cylindriques 97, d'axes longitudinaux, dans lesquels sont encastrées trois fiches métalliques femelles 99, à savoir deux fiches supérieures latérales et une fiche inférieure centrale sur la figure 5. Les fiches supérieures assurent la connexion électrique avec les pastilles piézo-électriques de la tête 71, et la fiche inférieure, éventuellement connectable, favorise dans le cas présent le centrage avec le connecteur d'irrigation 73.

La tête 71 est également en mesure de se connecter, de façon connue, avec l'élément d'alimentation 75. Celui-ci est constitué d'un corps cylindrique 101, en matière plastique, de même diamètre que le corps 77 de la tête 71, et dont la forme de la partie antérieure est complémentaire de celle de la partie postérieure de la tête 71. Ainsi, la face antérieure 103 de l'élément d'alimentation 75 est creusée d'une cavité cylindrique 105, d'un diamètre sensiblement égal à celui de la portion cylindrique 79, dans laquelle est formé un bossage cylindrique longitudinal 107, de section transversale semi-circulaire, complémentaire de celle du chambrage 87, et dans laquelle est creusé un logement cylindrique 109 d'un diamètre interne sensiblement égal à celui du tube 91 de façon à pouvoir recevoir celui-ci. L'élément d'alimentation 75 est percé d'un canal longitudinal 111, relié à des moyens d'alimentation (non représentés sur le dessin) en un premier liquide d'irrigation, qui débouche dans le fond du logement 109. Trois broches mâles longitudinales 113, complémentaires des fiches femelles 99, font saillie à la base de la cavité 105 et sont disposées de façon qu'elles puissent prendre place à l'intérieur des fiches femelles 99 de la tête 71, lorsque l'élément d'alimentation 75 est connecté à celle-ci. Les deux fiches femelles 99 supérieures sont reliées à des fils d'alimentation en courant électrique.

On constate que, cette connexion une fois établie, un premier liquide d'irrigation, qui arrive par le canal 111 de l'élément d'alimentation 75, peut s'écouler par le canal central 93 de la tête 71, vers l'extrémité antérieure de celle-ci, et que le circuit électrique de l'élément d'alimentation 75 est raccordé, par l'intermédiaire des broches mâles 113 et des fiches femelles 99 à la tête 71, et plus précisément aux pastilles piézo-électriques.

Dans une telle configuration de type connu, le praticien est en mesure d'effectuer des travaux d'odontologie de type classique tels que le détartrage de dents.

Pour effectuer une intervention, nécessitant à la fois un instrument et un liquide d'irrigation stériles, tel que, par exemple, un surfaçage radiculaire de la dent d'un patient, le praticien utilise, suivant l'invention, un connecteur d'irrigation 73, tel que celui représenté sur la figure 4b.

Ce connecteur d'irrigation 73 est mis en place dans la position représentée sur le dessin, c'est-à-dire entre la tête 71 et l'élément d'alimentation 75. A cet effet, il est essentiellement constitué d'un corps 117 dont la partie antérieure est identique à la partie antérieure de l'élément d'alimentation 75 et la partie postérieure est identique à la partie postérieure de la tête 71 (les éléments constitutifs de la partie antérieure et de la partie postérieure du connecteur d'irrigation 73 seront désignés par les mêmes références que celles utilisées respectivement pour la tête 71 et pour l'élément d'alimentation 75, affectées du signe '). Le connecteur d'irrigation 73 comprend ainsi une partie antérieure cylindrique creusée d'une cavité cylindrique 105' ouverte sur l'avant du connecteur et comportant des moyens de connexion hydrauliques constitués d'un bossage cylindrique longitudinal 107' de section semi-circulaire, creusé d'un logement cylindrique 109' en communication avec une canalisation radiale 119 reliée à des moyens d'alimentation en au moins un second liquide d'irrigation, et notamment un liquide d'irrigation stérile, et des moyens de connexion électrique constitués de trois broches mâles 113'. Le connecteur d'irrigation 73 comprend également une partie postérieure cylindrique 79', complémentaire de la cavité cylindrique 105', qui est creusée d'une cavité semi-cylindrique 87', complémentaire du bossage 107', à partir du fond de laquelle s'étend, vers l'arrière, un bossage cylindrique 91', complémentaire de la cavité 109', cet qui est dépourvu de canal central, de façon à se comporter comme un bouchon et à obturer ainsi de façon étanche, le logement 109 de l'élément d'alimentation 75, en empêchant de ce fait l'écoulement du premier liquide d'irrigation amené par le canal 111, lorsque le connecteur d'irrigation 73 est accouplé à l'élément d'alimentation 75. La partie postérieure du connecteur d'irrigation 73 comprend également des moyens de connexion électriques constitués de trois fiches femelles 99' complémentaires des broches mâles 113.

Dans ces conditions, lorsque le praticien souhaite effectuer une intervention nécessitant un matériel et un liquide stériles, il lui suffit alors de disposer un connecteur d'irrigation 73 suivant l'invention (stérile), raccordé à des moyens d'alimentation en liquide stérile, sur un élément d'alimentation 75 (non stérile), et de fixer une tête (stérile) 71 sur le connecteur d'irrigation 73, ce qui est une opération particulièrement facile et rapide à effectuer.

Comme représenté sur la figure 6, l'arrivée du second liquide d'irrigation dans la pièce à main ou dans le connecteur d'irrigation 73 peut se faire par l'intermédiaire d'un robinet de commande 121. Ce dernier est constitué, par exemple, d'un corps tubulaire 123 dont le fond est pourvu d'un orifice 125 qui est en communication, par un canal 127, avec le logement 109' du connecteur d'irrigation 73. L'arrivée du liquide d'irrigation dans le robinet 121 se fait par une canalisation 129 reliée à des moyens d'alimentation en liquide stérile, non représentés sur le dessin. Un piston 131, qui comporte une partie inférieure tronconique 133 qui pénètre dans l'orifice 125 de façon à en contrôler le débit en l'obturant plus ou moins, est monté à coulissement étanche dans ledit corps tubulaire 123. Ainsi, en enfonçant plus ou moins le piston 131 dans le corps tubulaire 123, on peut obturer plus ou moins l'orifice 125 et contrôler ainsi le débit du fluide d'irrigation admis dans le connecteur 73.

La présente invention ne permet pas seulement au praticien de passer rapidement d'une configuration non stérile à une configuration stérile mais, en utilisant plusieurs connecteurs d'irrigation et leurs liquides d'irrigation associés, elle permet également au praticien de disposer, au cours d'une même intervention, de différents liquides d'irrigation (qu'il peut éventuellement mélanger) auxquels il peut faire appel facilement et rapidement en fonction de ses besoins.

Afin de faciliter le passage d'un liquide d'irrigation donné à un autre, on pourrait utiliser, comme représenté sur la figure 7, une canalisation 119 comportant plusieurs conduits d'alimentation 135a, 135b aptes à être reliés à des réservoirs contenant des liquides d'irrigation différents. Les différents conduits 135a,135b peuvent être pourvus de moyens d'obturation, progressifs ou non, ce qui donne au praticien, s'il le souhaite, la possibilité de mélanger les différents liquides d'irrigation et de doser la proportion de leur mélange admis dans le connecteur 73.

De préférence, le raccordement des différents conduits d'irrigation 135a,135b s'effectue aussi près que possible du connecteur d'irrigation 73, ce qui présente l'avantage, lorsque l'on souhaite passer d'un premier liquide d'irrigation à un autre liquide d'irrigation différent, de n'avoir à évacuer qu'une quantité minimale du premier liquide d'irrigation.

Dans un mode de mise en oeuvre de l'invention, représenté sur la figure 8, l'obturation du canal 109 d'alimentation en un premier liquide, de l'élément d'alimentation 75, est assurée par un élément obturateur indépendant de l'élément actif, constitué d'un bouchon 200 qui est, par exemple, fixé par vissage dans ledit canal 109 avant la mise en place de l'élément actif sur l'élément d'alimentation 75. A cet effet la partie antérieure de celui-ci est pourvue d'une portion filetée 202. Le bouchon 200 comporte une tête filetée 204 apte à se visser dans cette portion filetée 202, prolongée, dans l'intérieur du canal 109, par une portion 206 de plus faible diamètre pourvue d'un joint torique 208 assurant l'étanchéité avec la paroi interne du canal 109. Dans une variante du présent mode de mise en oeuvre de l'invention le bouchon obturateur peut également être fixé au fond de la canalisation d'alimentation, ce qui permet de dégager la partie antérieure de celle-ci et lui permet ainsi de recevoir les tubes d'alimentation d'éléments actifs de type classique.

Bien entendu, les moyens de connexion hydrauliques et électriques ainsi que leur agencement pourraient être de tout type. On pourrait ainsi notamment disposer sur un même élément tous les connecteurs femelles et sur l'autre élément tous les connecteurs mâles, sans sortir du champ de protection de l'invention.

## Revendications

1. Instrument chirurgical et notamment instrument chirurgical alimenté en au moins un fluide stérile, comprenant un élément actif (1,50,70,71,73) et un élément d'alimentation (3,53,72,75), la partie postérieure de l'élément actif (1,50,70,71,73) et la partie antérieure de l'élément d'alimentation (3,53,72,75) comportant des moyens de connexion complémentaires permettant d'assurer leur solidarisation de façon étanche, l'élément d'alimentation (3,53,72,75) comportant au moins une canalisation d'alimentation (43, 68,68', 92,92', 96,96', 109) en un premier fluide, débouchant à l'endroit des moyens de connexion, et l'élément actif (1,50,70,71,73) comprend des moyens d'alimentation (25, 58,58', 80,80', 119, 129, 135a,135b) en au moins un second fluide, caractérisé en ce qu'il comporte des moyens (31, 64,64', 90,90', 91, 200) assurant l'obturation de la susdite canalisation d'alimentation (43, 68,68', 92,92', 96,96', 109) en un premier fluide, lorsque l'élément actif (1,50,70,71,73) est connecté à l'élément d'alimentation (3,53,72,75),

2. Instrument chirurgical suivant la revendication 1 caractérisé en ce que les moyens assurant l'obturation de la susdite canalisation d'alimentation (43, 68,68', 92,92', 96,96', 109) en un premier fluide, lorsque l'élément actif (1,50,70,71,73) est connecté à l'élément d'alimentation (3,53,72,75), font partie intégrante de la partie postérieure de l'élément actif (1,50,70,71,73).

3. Instrument chirurgical suivant l'une des revendications 1 ou 2 dans lequel l'élément d'alimentation (3,53,72,75) comporte des moyens d'alimentation en énergie, caractérisé en ce que lesdits moyens de connexion assurent le transfert de l'énergie de l'élément d'alimentation (3,53,72,75) vers l'élément actif (1,50,70).

4. Instrument chirurgical suivant la revendication 3 caractérisé en ce que les moyens d'alimentation en énergie sont des moyens d'alimentation en énergie électrique.

5. Instrument chirurgical suivant la revendication 3 caractérisé en ce que les moyens d'alimentation en énergie sont des moyens d'alimentation en énergie pneumatique.

6. Instrument chirurgical suivant l'une quelconque des revendications précédentes caractérisé en ce que
- l'élément actif est constitué d'au moins deux éléments (71,73) connectables entre eux de façon étanche, à savoir un élément antérieur, ou tête (71), et un élément postérieur, ou connecteur d'irrigation (73),
- les moyens d'alimentation (119) en au moins un second fluide sont en communication avec le connecteur d'irrigation (73),
- la partie postérieure de la tête (71) comporte des moyens de connexion complémentaires de ceux de l'élément d'alimentation (75) permettant d'assurer une solidarisation étanche avec celui-ci, le canal d'alimentation (109) en un premier fluide étant en communication avec la tête (71) lorsque cette dernière est connectée à l'élément d'alimentation (75).

7. Instrument chirurgical suivant l'une quelconque des revendications précédentes, caractérisé en ce que les moyens d'alimentation en au moins un second fluide sont constitués d'au moins un tube d'alimentation (25,58,58',80,80', 119,129,135a,135b), solidaire de l'élément actif, en communication avec une source externe de fluide.

8. Instrument chirurgical suivant la revendication 7 caractérisé en ce que ledit tube d'alimentation (119) est en communication avec au moins deux autres tubes (135a, 135b) reliés chacun à une source externe de fluide.

9. Instrument chirurgical suivant la revendication 8 caractérisé en ce que le tube d'alimentation (119) et les susdits deux autres tubes (135a, 135b) se rencontrent à proximité de l'élément actif.

10. Instrument chirurgical suivant la revendication 7 caractérisé en ce que le tube d'alimentation (119) et/ou l'un au moins des susdits autres tubes (135a, 135b) est muni de moyens de contrôle du débit du second fluide admis dans l'élément actif.

## Claims

1. Surgical instrument and in particular a surgical instrument supplied with at least one sterile fluid, comprising an active element (1, 50, 70, 71, 73) and a supply element (3, 53, 72, 75), the rear part of the active element (1, 50, 70, 71, 73) and the front part of the supply element (3, 53, 72, 75) comprising complementary connectors ensuring sealed connection thereof, the supply element (3, 53, 72, 75) comprising at least one duct (43, 68, 68', 92, 92', 96, 96', 109) supplying a first fluid, opening at the connectors, and the active element (1, 50, 70, 71, 73) comprises means (25, 58, 58', 80, 80', 119, 129, 135a, 135b) for supplying at least one second fluid, characterized in that it comprises means (31, 64, 64', 90, 90', 91, 200) for obturating said duct (43, 68, 68', 92, 92', 96, 96', 109) supplying a first fluid, when the active element (1, 50, 70, 71, 73) is connected to the supply element (3, 53, 72, 75).

2. Surgical instrument according to Claim 1, characterized in that the means for obturating said duct (43, 68, 68', 92, 92', 96, 96', 109) supplying a first fluid, when the active element (1, 50, 70, 71, 73) is connected to the supply element (3, 53, 72, 75), are an integral part of the rear part of the active element (1, 50, 70, 71, 73).

3. Surgical instrument according to one of Claims 1 or 2 in which the supply element (3, 53, 72, 75) comprises energy supply means, characterized in that said connectors ensure transfer of the energy from the supply element (3, 53, 72, 75) towards the active element (1, 50, 70).

4. Surgical instrument according to Claim 3, characterized in that the energy supply means are means supplying electrical energy.

5. Surgical instrument according to Claim 3, characterized in that the energy supply means are means supplying pneumatic energy.

6. Surgical instrument according to any one of the preceding Claims, characterized in that
- the active element is constituted by at least two elements (71, 73) connectable together in sealed manner, namely a front element, or head (71), and a rear element, or irrigation connector (73),
- the means (119) for supplying at least one second fluid are in communication with the irrigation connector (73),
- the rear part of the head (71) comprises connectors complementary of those of the supply element (75) ensuring sealed connection therewith, the duct (109) supplying a first fluid being in communication with the head (71) when the latter is connected to the supply element (75).

7. Surgical instrument according to any one of the preceding Claims, characterized in that the means for supplying at least one second fluid are constituted by at least one supply tube (25, 58, 58', 80, 80', 119, 129, 135a, 135b) fast with the active element, in communication with an outside source of fluid.

8. Surgical instrument according to Claim 7, characterized in that said supply tube (119) is in communication with at least two other tubes (135a, 135b) each connected to an outside source of fluid.

9. Surgical instrument according to Claim 8, characterized in that the supply tube (119) and the said other two tubes (135a, 135b) meet one another in the vicinity of the active element.

10. Surgical instrument according to Claim 7, characterized in that the supply tube (119) and/or at least one of said other tubes (135a, 135b) is provided with means for controlling the flowrate of the second fluid admitted into the active element.

## Patentansprüche

1. Chirurgisches Instrument und insbesondere ein solches, dem mindestens ein steriles Fluid zugeführt wird, mit:
mindestens einem aktiven Element (1, 50, 70, 71, 73) und einem Versorgungselement (3, 53, 72, 75), wobei der hintere Teil des aktiven Elements (1, 50, 70, 71, 73) und der vordere Teil des Versorgungselements (3, 53, 72, 75) komplementäre Verbindungseinrichtungen aufweisen, die in dichtender Weise fest verbunden werden können, und das Versorgungselement (3, 53, 72, 75) mindestens eine Leitung (43, 68, 68', 92, 92', 96, 96', 109) zur Versorgung mit einem ersten Fluid aufweist, die an der Stelle der Verbindungseinrichtungen einmündet, und das aktive Element (1, 50, 70, 71, 73) Einrichtungen (25, 58, 58', 80, 80', 119, 129, 135a, 135b) zur Versorgung mit mindestens einem zweiten Fluid aufweist,
**gekennzeichnet durch**
Einrichtungen (31, 64, 64', 90, 90', 91, 200), welche die Leitung (43, 68, 68', 92, 92', 96, 96', 109) zur Versorgung mit einem ersten Fluid abdichten, wenn das aktive Element (1, 50, 70, 71, 73) mit dem Versorgungselement (3, 53, 72, 75) verbunden ist.

2. Chirurgisches Instrument nach Anspruch, 1, dadurch gekennzeichnet, daß die Einrichtungen, welche die Leitung (43, 68, 68', 92, 92', 96, 96', 109) zur Versorgung mit einem ersten Fluid abdichten, wenn das aktive Element (1, 50, 70, 71, 73) mit dem Versorgungselement (3, 53, 72, 75) verbunden ist, integraler Teil des hinteren Teils des aktiven Elements (1, 50, 70, 71, 73) sind.

3. Chirurgisches Instrument nach einem der Ansprüche 1 oder 2, bei dem das Versorgungselement (3, 53, 72, 75) Einrichtungen zur Versorgung mit Energie aufweist, dadurch gekennzeichnet, daß die Verbindungseinrichtungen Energie vom Versorgungselement (3, 53, 72, 75) zum aktiven Element (1, 50, 70) übertragen.

4. Chirurgisches Instrument nach Anspruch 3, dadurch gekennzeichnet, daß die Einrichtungen zur Versorgung mit Energie Einrichtungen zur Versorgung mit elektrischer Energie sind.

5. Chirurgisches Instrument nach Anspruch 3, dadurch gekennzeichnet, daß die Einrichtungen zur Versorgung mit Energie Einrichtungen zur Versorgung mit pneumatischer Energie sind.

6. Chirurgisches Instrument nach einem der vorstehenden Ansprüche, dadurch gekennzeichnet, daß
- das aktive Element aus mindestens zwei Elementen (71, 73), die miteinander in dichtender Weise verbunden werden können, besteht, und zwar einem vorderen Element, oder Kopf (71), und einem hinteren Element, oder Einström-Verbindungsstück (73),
- die Einrichtungen (119) zur Versorgung mit mindestens einem zweiten Fluid in Verbindung mit dem Einström-Verbindungsstück (73) stehen,
- der hintere Teil des Kopfs (71) Verbindungseinrichtungen aufweist, die zu denen des Versorgungselements (75) komplementär sind und eine dichtende Verbindung mit diesem erlauben, wobei die Leitung (109) zur Versorgung mit einem ersten Fluid in Verbindung mit dem Kopf (71) steht, wenn dieser mit dem Versorgungselement (75) verbunden ist.

7. Chirurgisches Instrument nach einem der vorstehenden Ansprüche, dadurch gekennzeichnet, daß die Einrichtungen zur Versorgung mit mindestens einem zweiten Fluid aus mindestens einem zweiten Versorgungsrohr (25, 58, 58', 80, 80', 119, 129, 135a, 135b) bestehen, das mit dem aktiven Element fest verbunden ist und mit einer externen Fluidquelle in Verbindung steht.

8. Chirurgisches Instrument nach Anspruch 7, dadurch gekennzeichnet, daß das Versorgungsrohr (119) in Verbindung mit mindestens zwei weiteren Rohren (135a, 135b) steht, von denen jedes mit einer externen Fluidquelle verbunden ist.

9. Chirurgisches Instrument nach Anspruch 8, dadurch gekennzeichnet, daß das Versorgungsrohr (119) und die zwei weiteren Rohre (135a, 135b) in der Nähe des aktiven Elements aufeinander treffen.

10. Chirurgisches Instrument nach Anspruch 7, dadurch gekennzeichnet, daß das Versorgungsrohr (119) und/oder mindestens eines der weiteren Rohre (135a, 135b) mit Einrichtungen zur Regelung des Durchsatzes von dem in das aktive Element einströmenden zweiten Fluid versehen ist.
